(19) Europäisches Patentamt / European Patent Office / Office européen des brevets

(11) **EP 2 677 920 B1**

(12) **FASCICULE DE BREVET EUROPEEN**

(45) Date de publication et mention
de la délivrance du brevet:
**03.04.2019 Bulletin 2019/14**

(21) Numéro de dépôt: **12707722.0**

(22) Date de dépôt: **21.02.2012**

(51) Int Cl.:
*A61B 3/00* (2006.01)   *A61B 3/14* (2006.01)
*A61B 3/10* (2006.01)   *A61B 3/15* (2006.01)

(86) Numéro de dépôt international:
**PCT/EP2012/052933**

(87) Numéro de publication internationale:
**WO 2012/113790 (30.08.2012 Gazette 2012/35)**

(54) **MÉTHODE ET DISPOSITIF D'IMAGERIE RÉTINIENNE A HAUTE RÉSOLUTION**

VERFAHREN UND VORRICHTUNG FÜR HOCHAUFLÖSENDE RETINABILDGEBUNG

METHOD AND DEVICE FOR HIGH-RESOLUTION RETINAL IMAGING

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **22.02.2011 FR 1151440**

(43) Date de publication de la demande:
**01.01.2014 Bulletin 2014/01**

(73) Titulaire: **Imagine Eyes**
**91400 Orsay (FR)**

(72) Inventeur: **LEVECQ, Xavier**
**91190 Gif Sur Yvette (FR)**

(74) Mandataire: **Osha Liang**
**2, rue de la Paix**
**75002 Paris (FR)**

(56) Documents cités:
**EP-A1- 1 561 416    WO-A1-2010/079550
WO-A1-2010/083381    WO-A1-2010/128651
WO-A2-01/58339    WO-A2-2007/035334
US-A1- 2003 058 403    US-A1- 2007 258 045**

## Description

### Domaine technique de l'invention

La présente invention concerne une méthode et un dispositif d'imagerie rétinienne, présentant une résolution compatible avec l'imagerie à l'échelle cellulaire, et basés sur l'utilisation de l'optique adaptative.

### Etat de l'art

Il s'écoule plusieurs années aujourd'hui entre le début d'une maladie rétinienne et son diagnostic. En effet, les maladies rétiniennes en général se développent silencieusement en provoquant des lésions irréversibles avant que les premiers symptômes cliniques apparaissent. C'est le cas par exemple de la Dégénérescence Maculaire Liée à l'Age (ou DMLA) ou du glaucome, maladie s'attaquant aux fibres nerveuses de la rétine et pouvant provoquer la cécité du sujet, et qui est généralement diagnostiquée lorsque la moitié des fibres nerveuses sont irrémédiablement détruites. Or les maladies rétiniennes peuvent être diagnostiquées dès les premières semaines si on peut imager la rétine à l'échelle cellulaire. En effet, les premiers effets des maladies rétiniennes atteignent les structures microscopiques de la rétine. Les microstructures atteintes par les 3 maladies rétiniennes les plus courantes et parmi les plus graves (DMLA, glaucome, rétinopathie diabétique) sont les photorécepteurs, dont les cônes, cellules photosensibles qui détectent la lumière et qui présentent une taille variant entre 2 et 5 $\mu$m, les micro vaisseaux de la rétine qui sont les plus petit vaisseaux du corps humain (environ 6 $\mu$m de diamètre), et les faisceaux de fibres nerveuses qui présentent quant à eux un diamètre d'environ 10 $\mu$m.

De nombreux laboratoires travaillent sur différentes technologies qui permettraient de faire de l'imagerie rétinienne avec une résolution cellulaire. Ces différentes technologies proposent des systèmes d'illumination et/ou détection de la rétine différents mais toutes mettent en oeuvre un système d'optique adaptative permettant de mesurer les défauts optiques de l'oeil et du système d'imagerie et de corriger les rayons lumineux issus de la rétine et incidents sur le système de détection pour gagner en résolution.

Parmi ces technologies, on peut citer l'ophtalmoscopie à balayage laser avec optique adaptative ou 'AOSLO' (abréviation de l'expression anglo-saxonne 'Adaptive Optics Scanning Laser Ophthalmoscopy'). Un montage d'AOSLO est par exemple décrit dans A. Roorda et al. (« Adaptive optics scanning laser ophthalmoscopy », Optics express 405, Vol. 10, N°9, 2002). Le montage de l'AOSLO comprend principalement un système d'illumination de la rétine, un dispositif de détection, un système de balayage (« scanning »), un système de correction comprenant un plan de correction d'ondes lumineuses incidentes, un système de mesure des défauts optiques comprenant un plan d'analyse des défauts optiques de rayons lumineux incidents, et une optique d'imagerie. Le système d'illumination comprend par exemple une diode laser couplée à une fibre optique pour former un point source et une lentille optique permettant de former à partir du point source un faisceau d'éclairage. Le faisceau d'éclairage est envoyé, par exemple par un jeu de miroirs, sur le système de correction, par exemple un miroir déformable, puis dans le système de balayage pour être dirigé selon un balayage vertical et horizontal dans l'oeil du sujet. Le faisceau d'éclairage est ainsi focalisé pour former sur la rétine un faisceau quasi ponctuel qui balaye la rétine et la lumière rétrodiffusée par la rétine subit le même balayage optique au retour pour être envoyée sur le miroir déformable et dispositif de détection, comprenant par exemple un trou de détection confocale et un détecteur qui peut être un photomultiplicateur ou une photodiode à avalanche. Un ensemble d'éléments optiques permet de conjuguer optiquement le plan de la rétine et le plan de détection qui est celui du trou de détection confocale du détecteur. Le système de mesure des défauts optiques comprend par exemple un analyseur de type Shack-Hartmann ; il reçoit la lumière rétrodiffusée par la rétine et contrôle le miroir déformable afin de corriger le faisceau d'éclairage et le faisceau rétrodiffusé. Le système d'illumination peut comprendre une première source d'émission d'un faisceau pour l'imagerie et une seconde source d'émission d'un faisceau pour l'analyse des défauts optiques distincte de la source d'imagerie, ou ces deux sources peuvent être confondues, le même faisceau d'éclairage de la rétine servant pour l'imagerie et l'analyse.

On connaît également les montages de type OCT (abréviation de l'expression anglo-saxonne 'Optical Coherent Tomography') couplés à de l'optique adaptative. Un tel système est décrit par exemple dans R. Zawadzki (« Adaptive-optics optical coherence tomography for high resolution and high speed 3D retinal in vivo imaging » Optics Express 8532, Vol. 13, N°21, 2005). L'OCT repose sur l'utilisation d'un interféromètre à faible cohérence. Cette technique d'imagerie permet de réaliser in vivo des images en coupe de tissus, avec une résolution de quelques microns. Les montages de type OCT comprennent un arrangement similaire à celui de l'AOSLO mais dans lequel le système de détection est spécifique à l'OCT et comprend notamment un interféromètre, par exemple un interféromètre fibré, par exemple de type Michelson. Le plan de détection est le plan comprenant le point d'entrée de la fibre ; il est conjugué avec la rétine de l'oeil au moyen d'un système optique de conjugaison. Un intérêt de l'OCT en ophtalmologie provient de sa capacité à révéler in-vivo des tissus au travers d'autres tissus diffusants. En comparaison avec l'AOSLO, la technologie OCT permet d'avoir une coupe longitudinale de la rétine au détriment de la vitesse d'acquisition.

Une troisième technique connue est l'imagerie rétinienne plein champ ou 'flood' selon l'expression anglo-saxonne, décrit par exemple dans H. Hofer et al. (« Improvements in retinal image quality with dynamic correction of the eye's aberrations », Optics Express, Vol. 8, Issue 11, pp. 631-643, 2001) ou dans A. Roorda ("Adaptive Optics Ophthalmoscopy", Journal of refractive Surgery, Vol. 16, 2000). Par rapport aux systèmes de type OCT ou AOSLO, les dispositifs d'imagerie rétinienne de type 'flood' sont limités dans l'exploration en profondeur de la rétine, mais présentent l'avantage de fonctionner en plein champ, c'est-à-dire sans balayage mécanique de la rétine, et avec des temps d'acquisition de l'image complète beaucoup plus court, ce qui les rend à la fois moins complexes à réaliser, moins coûteux et moins sensibles aux déformations que subit l'image pendant le temps d'acquisition, déformations qui sont générées par le mouvement de la rétine.

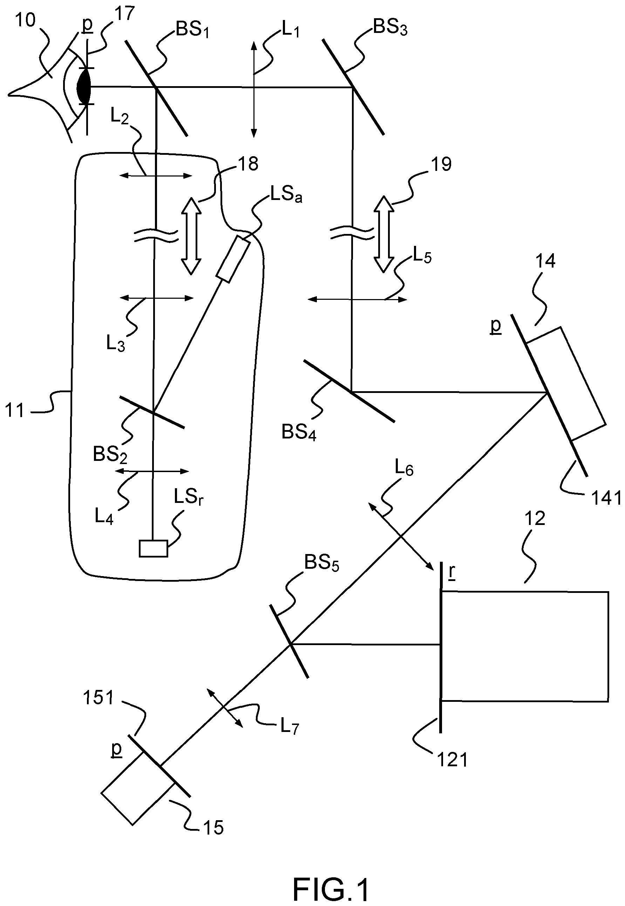

Un montage d'imagerie rétinienne de type plein champ selon l'art antérieur est décrit sur la figure 1. Il comprend de façon générale un bloc illumination 11 avec une première source d'illumination $LS_r$ pour l'imagerie et une seconde source d'illumination $LS_a$ pour l'analyse des défauts optiques. Il comprend également un bloc détecteur 12 avec un dispositif d'acquisition multidétecteur (ou détecteur matriciel), par exemple une camera CCD, dont le plan de détection 121 est destiné à être conjugué optiquement avec la rétine de l'oeil 10 que l'on cherche à imager, grâce à un système optique de formation d'image - ou système d'imagerie - regroupant dans cet exemple un ensemble d'objectifs $L_1$, $L_5$, $L_6$ et des lames séparatrices. Le système d'imagerie forme avec le détecteur la voie d'imagerie. La rétine est destinée à être éclairée selon le champ requis par les faisceaux issus de la source d'imagerie $LS_r$ et envoyés sur la rétine au moyen d'un ensemble d'objectifs $L_4$, $L_3$, $L_2$ et de la lame séparatrice $BS_1$. Un dispositif de correction 14, par exemple un miroir déformable, comprenant un plan de correction 141 des rayons lumineux rétrodiffusées par la rétine, contrôlé par un système de mesure des défauts optiques 15, permet de corriger tout ou partie des défauts optiques dus à l'oeil et au système optique du système d'imagerie et d'améliorer ainsi la qualité de l'image de la rétine formée sur le bloc de détection 12. Le système de mesure des défauts optiques permet de déterminer dans un plan d'analyse et en une mesure les défauts optiques d'une onde lumineuse incidente. Dans la suite de la description, on entend par « défauts optiques » l'ensemble des perturbations que subissent les rayons lumineux entre la rétine et le plan d'analyse. Ces défauts comprennent notamment les défauts apportés par le système optique de l'oeil mais aussi ceux apportés par la partie du système d'imagerie commune avec la voie d'analyse. Le système de mesure des défauts optiques 15 est avantageusement un analyseur de type Shack-Hartmann comprenant un plan d'analyse 151 formé d'un ensemble de microlentilles et d'un détecteur matriciel agencé dans le plan focal desdites microlentilles. Il reçoit une onde issue de la source d'illumination ponctuelle $LS_a$, focalisée sur la rétine au moyen d'un ensemble d'objectifs optiques $L_2$, $L_4$ et de lames séparatrices $BS_2$, $BS_1$ pour former un point source secondaire, puis rétrodiffusée vers le système d'analyse. Dans ce système, le plan d'analyse de l'analyseur des défauts optiques et le plan de correction du dispositif de correction sont conjugués optiquement avec un plan prédéterminé dans l'espace d'entrée du système d'imagerie, plan réel destiné à être confondu avec un plan prédéterminé de l'oeil, par exemple le plan pupillaire de l'oeil. La pupille d'entrée du système d'imagerie est avantageusement située dans ce même plan prédéterminé dans l'espace d'entrée du système d'imagerie. La voie d'analyse est ainsi formée du système de mesure des défauts optiques 15 et des moyens de conjugaison du plan d'analyse avec ledit plan prédéterminé dans l'espace d'entrée du système d'imagerie. La pupille d'entrée du système d'imagerie est par exemple une image de la pupille physique du dispositif de correction, formée par exemple par un diaphragme et définissant la surface utile du dispositif de correction. De manière générale, on entend par « pupille d'entrée » d'un système optique, la plus petite ouverture qui limite l'entrée ou la propagation des rayons lumineux dans le système. Cette ouverture peut être réelle dans le cas où un diaphragme physique, pupille du système optique considéré, limite l'entrée des rayons lumineux ou virtuelle dans le cas où cette ouverture est une image de la pupille physique du système optique qui se trouve à l'intérieur du système optique et qui est formé par exemple par un diaphragme. Ainsi, dans le cas où la pupille d'entrée du système d'imagerie de la rétine est positionnée dans le plan pupillaire de l'oeil ou dans un plan se situant à proximité de ce dernier, ladite pupille d'entrée est virtuelle, image d'un diaphragme physique situé à l'intérieur dudit système optique d'imagerie. Comme cela est illustré sur la figure 1, on cherche dans un système à haute résolution à rendre maximal le trajet optique commun entre la voie d'analyse et la voie d'imagerie pour limiter au maximum les défauts optiques différentiels entre ces deux voies et éviter de réinjecter dans la correction des défauts optiques situés dans la voie d'analyse (non présents dans la voie d'imagerie) et de ne pas corriger des défauts optiques situés dans la voie d'imagerie (non présents dans la voie d'analyse).

Un dispositif d'imagerie rétinienne de type plein champ tel que décrit sur la figure 1 voit cependant sa résolution limitée du fait des réflexions parasites sur la cornée. En effet, la réflexion cornéenne due aux réflexions sur la cornée des faisceaux issus de la source d'illumination d'imagerie ajoute au signal lumineux utile un fond continu au niveau du plan pupillaire qui entraîne du bruit dans le système de détection, bruit qui contribue à la détérioration du rapport signal à bruit et donc à la dégradation de la qualité de l'image. Compte tenu du fait que cette réflexion cornéenne ce situe dans un plan proche du plan pupillaire et donc éloigné du plan d'imagerie, cette réflexion amène sur le détecteur un flux lumineux réparti de façon relativement uniforme. Ce flux parasite engendre un bruit de photon supplémentaire qui détériore le rapport signal à bruit de l'image réalisée. A titre d'exemple, le coefficient de réflexion de la cornée est donné par la formule $(n_c-1)^2/(n_c+1)^2$ où $n_c$ est l'indice de réfraction du film de larme sur la cornée, soit 1.3 environ. Le coefficient de réflexion est donc de 1,7%. Or le rapport entre le flux récupéré comportant le signal utile et le flux envoyé dans l'oeil par la source d'imagerie se situe entre 1/50000 et 1/100000. Pour optimiser la qualité de l'image il est donc nécessaire de réduire au maximum ce flux parasite lié à la réflexion cornéenne.

Le blocage des réflexions cornéennes est une problématique sur laquelle s'est penché l'homme de l'art. Notamment, il a été proposé pour l'imagerie un éclairage annulaire en périphérie de la pupille oculaire (voir par exemple le brevet US3594071 dans le domaine des caméras pour l'imagerie du fond de l'oeil). Dans cette configuration, la pupille d'entrée du système d'imagerie est centrée au milieu de la pupille oculaire et la réflexion cornéenne ne perturbe pas l'imagerie. Il est également possible d'envisager un éclairage hors axe sur la voie d'analyse afin d'éviter d'éclairer le sommet de la rétine, qui, couplé à un trou de filtrage dans le plan de la rétine, permet de bloquer les réflexions cornéennes.

Cependant ni l'une ni l'autre de ces approches ne peuvent être adoptées pour l'imagerie à haute résolution de la couche des photorécepteurs de la rétine. En effet pour obtenir un bon contraste sur les cônes (photorécepteurs) il faut optimiser le système d'imagerie en tenant compte de l'effet Styles Crawford, comme cela est décrit par exemple dans A. Roorda ("Adaptive Optics Ophthalmoscopy", Journal of refractive Surgery, Vol. 16, 2000). Cet effet, lié à la géométrie des cônes photorécepteurs, fait que leur réflectivité dépend de l'angle d'incidence des rayons lumineux qui éclairent la rétine. Ainsi, on cherche pour l'optimisation du système d'imagerie, à ce que l'angle d'arrivée des rayons sur les photorécepteurs soit le plus faible possible. L'éclairage annulaire tel qu'utilisé dans l'art antérieur entraîne un angle d'incidence important des rayons sur les photorécepteurs ce qui ne permet pas d'obtenir l'effet Styles Crawford. Au contraire, l'optimisation de l'effet Styles Crawford impose un éclairage sensiblement centré sur le sommet de la cornée de façon à réduire au maximum l'angle d'incidence des rayons lumineux sur la couche des photorécepteurs. Ainsi l'imagerie à haute résolution de la rétine nécessite un éclairage centré sur l'axe optique de l'oeil. Par ailleurs, l'éclairage hors axe sur la voie d'analyse, outre le fait qu'elle n'optimise pas non plus l'effet Stiles Crawford, nécessite un filtrage spatial dans un plan conjugué du plan de la rétine pour bloquer la réflexion cornéenne, ce qui risque de limiter le champ imagé de la rétine si ce filtrage est effectué sur une partie commune des voies d'imagerie et d'analyse, partie commune que l'on cherche à rendre maximale. Le document WO 2010/128651 décrit un système d'imagerie rétinienne multi-faisceaux comprenant un dispositif de mesure de front d'onde avec un analyseur de type Shack-Hartman. Le document WO 2010/079550 décrit une unité OCT comprenant une voie d'imagerie rétinienne.

Le document US 2007/0258045 décrit un appareil d'imagerie ophtalmologique dans lequel un miroir percé est agencé pour couper sur une voie d'imagerie à forte résolution une partie des réflexions parasites issues du sommet de la cornée. Cependant, le système tel que divulgué dans ce document présente nécessairement des erreurs dans la mesure des défauts optiques par l'analyseur de front d'onde dues aux réflexions parasites sur la voie d'analyse, erreurs qui vont entacher la qualité de l'image.

Un objet de l'invention est de proposer un dispositif d'imagerie rétinienne qui supprime ou diminue fortement l'influence des réflexions cornéennes à la fois dans le plan d'imagerie et dans un plan proche d'une image du plan pupillaire de l'oeil sans entrainer de disfonctionnement ni de perte de performances. Notamment un objet de la présente invention est de prendre en compte la dimension nouvelle d'optique adaptative (c'est à dire de correction des défauts optiques) qui se pose dans une caméra rétinienne à haute résolution.

RESUME DE L'INVENTION

Selon un premier aspect, l'invention concerne un dispositif d'imagerie rétinienne comprenant :

- Au moins une source d'émission d'un faisceau lumineux pour l'illumination de la rétine d'un oeil d'un sujet,
- une voie d'imagerie de la rétine comprenant un dispositif de détection avec un plan de détection et un système optique d'imagerie,
- une voie d'analyse comprenant un dispositif de mesure de défauts optiques avec un plan d'analyse destiné à recevoir un ensemble de rayons lumineux rétrodiffusés par la rétine et des moyens de conjugaison optique dudit plan d'analyse avec un plan prédéterminé dans l'espace d'entrée du système d'imagerie de la voie d'imagerie,
- un dispositif de correction commun auxdites voies d'analyse et d'imagerie, comprenant un plan de correction et destiné à corriger dans ledit plan de correction les rayons lumineux issus de ladite source d'émission et rétrodiffusés par la rétine en fonction des défauts optiques mesurés par le dispositif de mesure,
- un système d'occultation de la lumière, positionné dans un plan voisin ou confondu avec ledit plan de correction ou dans un plan image dudit plan de correction situé sur un trajet optique commun des voies d'analyse et d'imagerie, et dimensionné pour occulter au moins partiellement les réflexions par la cornée des rayons lumineux issus de ladite source d'émission.

La déposante a montré qu'en agençant un système d'occultation dans le plan de correction, ou dans un plan conjugué du plan de correction si ce plan conjugué est sur un trajet optique commun des voies d'analyse et d'imagerie, on pouvait diminuer considérablement l'influence des réflexions à la fois pour l'imagerie et l'analyse, sans dégrader les performances du système ; tout au contraire, les performances du système se trouvent améliorées du fait du meilleur rapport signal sur bruit obtenu dans les deux voies.

Avantageusement, le plan de correction est lui-même conjugué du plan prédéterminé dans l'espace d'entrée du système d'imagerie de la voie d'imagerie, ledit plan prédéterminé étant par exemple le plan de la pupille d'entrée du système d'imagerie.

Avantageusement, le système d'occultation présente la forme d'un disque opaque, dont le diamètre, ramené dans l'espace d'entrée du système d'imagerie, est supérieur ou égal à une valeur d donnée par $d = \tan \theta \times R/2$ où R est le rayon de courbure de la cornée et $\theta$ la taille angulaire de la source d'émission.

Avantageusement, le système d'occultation est centré sur l'axe optique du système optique d'imagerie. Cette configuration est la plus avantageuse, notamment quand le centre de la cornée est centré sur la pupille de l'oeil, que l'on cherchera à centrer sur la pupille d'entrée du système optique d'imagerie.

Selon un exemple, le dispositif de correction comprend un miroir déformable. Alternativement, une valve à cristaux liquides peut être utilisée.

Selon un exemple le dispositif de mesure des défauts optiques est un analyseur de type Shack-Hartmann.

Avantageusement, le dispositif d'imagerie rétinienne comprend en outre un système de positionnement dans l'espace du dispositif d'imagerie par rapport à l'oeil, permettant d'assurer le centrage du système d'occultation avec l'image formée par le dioptre cornéen de la source d'imagerie.

Par exemple, le dispositif d'imagerie rétinienne est de type plein champ, comprenant une première source lumineuse d'imagerie pour l'illumination de la rétine avec un champ donné et une seconde source lumineuse d'analyse pour l'illumination de la rétine aux fins de l'analyse des défauts optiques par ledit dispositif de mesure des défauts optiques, le dispositif de détection comprenant un détecteur matriciel et le système d'occultation étant dimensionné pour occulter au moins partiellement les réflexions par le dioptre cornéen des rayons lumineux issus desdites sources.

Selon un second aspect, l'invention concerne une méthode d'imagerie rétinienne, comprenant :

- l'émission d'au moins un faisceau lumineux pour l'illumination de la rétine d'un oeil d'un sujet au moyen d'une source d'émission lumineuse,
- la formation d'une image d'au moins une partie de la rétine sur un plan de détection d'un dispositif de détection, au moyen d'un système optique d'imagerie définissant une voie d'imagerie,
- la mesure de défauts optiques par l'analyse dans un plan d'analyse donné des défauts optiques de rayons lumineux rétrodiffusés par la rétine, ledit plan d'analyse étant conjugué avec un plan prédéterminé de l'oeil au moyen d'un système de conjugaison optique définissant une voie d'analyse,
- la correction dans un plan de correction donné des rayons lumineux issus de ladite source d'émission et rétrodiffusés par la rétine en fonction des défauts optiques mesurés,
- l'occultation au moins partielle des réflexions par le dioptre cornéen des rayons lumineux issus de ladite source d'émission par un système d'occultation du flux lumineux agencé dans un plan voisin du plan de correction ou dans un plan conjugué du plan de correction situé sur un trajet commun aux voies d'analyse et d'imagerie.

Avantageusement, le plan de correction est conjugué avec ledit plan prédéterminé de l'oeil et ledit plan prédéterminé de l'oeil est sensiblement confondu avec le plan image de ladite source d'imagerie par le dioptre cornée, ce qui permet de minimiser la taille nécessaire de la zone d'occultation pour occulter les rayons réfléchis par le dioptre cornéen, issus de la source d'imagerie.

Avantageusement, ledit plan prédéterminé de l'oeil est le plan de la pupille d'entrée du système d'imagerie.

Avantageusement, les faisceaux issus de la ou desdites source(s) d'illumination de la rétine sont incidents dans l'oeil par le sommet de la cornée de l'oeil, permettant ainsi l'optimisation de l'effet Stiles Crawford.

Avantageusement, la méthode d'imagerie rétinienne comprend en outre le positionnement dans l'espace du système d'imagerie par rapport à l'oeil.

Selon une variante, la méthode d'imagerie rétinienne est de type plein champ et comprend l'émission d'un premier faisceau lumineux d'imagerie pour l'illumination de la rétine avec un champ donné et l'émission d'un second faisceau lumineux pour l'illumination de la rétine pour l'analyse des défauts optiques et l'occultation au moins partielle des réflexions par le dioptre cornéen desdits faisceaux lumineux d'imagerie et d'analyse par ledit système d'occultation.

BREVE DESCRIPTION DES DESSINS

D'autres avantages et caractéristiques de l'invention apparaîtront à la lecture de la description, illustrée par les figures suivantes :

- Figure 1 (déjà décrite), un système d'imagerie rétinienne de type plein champ selon de l'art antérieur ;

- Figure 2, un exemple de réalisation d'un système d'imagerie rétinienne de type plein champ selon l'invention;

- Figure 3, un schéma illustrant le calcul de la taille de l'image de la source d'imagerie par le dioptre cornéen ;

- Figure 4, un schéma illustrant un système d'alignement pour la mise en oeuvre de l'invention selon un exemple de réalisation;

- Figures 5A, 5B deux schémas montrant un exemple de procédure d'alignement mise en oeuvre par le système de la figure 4 ;

- Figure 6, une courbe montrant la comparaison de la FTM calculée en fonction de la fréquence dans un système selon l'art antérieur et deux exemples de système selon l'invention;

- Figure 7A et 7B des images de rétines mesurées dans des systèmes d'imagerie rétinienne de type plein champ à 850 nm, respectivement avec un système selon l'art antérieur et un système selon l'invention.

Par soucis de cohérence, les éléments identiques sont repérés par les mêmes références dans les différentes figures.

DESCRIPTION DETAILLEE

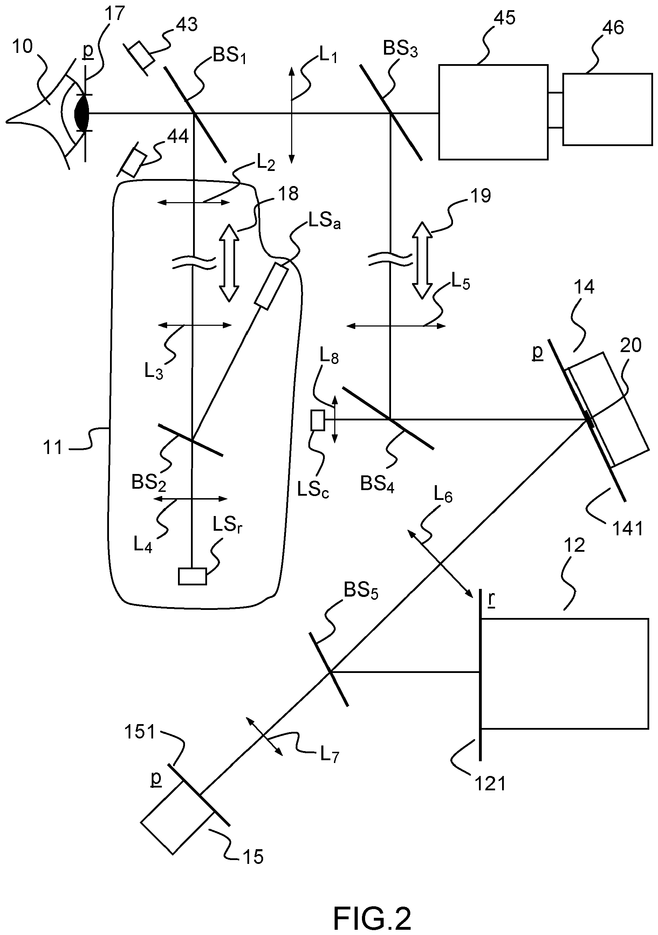

La figure 2 représente un exemple de dispositif d'imagerie rétinienne à haute résolution selon un exemple de réalisation de l'invention. Sur la figure 2, seuls les éléments du dispositif nécessaires à la compréhension de l'invention sont représentés. Le dispositif d'imagerie comprend un bloc illumination 11, avec une première source $LS_r$ d'émission d'un faisceau lumineux destiné à éclairer la rétine d'un oeil 10 d'un sujet pour en former une image au moyen du bloc détecteur 12. Cette source permet d'éclairer la rétine de l'oeil avec un champ θ donné, typiquement 4° x 4° pour former une image dite « plein champ ». Avantageusement, la source d'illumination de la rétine $LS_r$ présente une bande spectrale dans le proche infra rouge, typiquement entre 750 et 1100 nm, plage de longueurs d'onde présentant pour le sujet un confort oculaire plus grand et pour laquelle la longueur de pénétration dans les couches de la rétine est plus importante. Selon une variante, la bande spectrale de la source d'illumination de la rétine $LS_r$ peut également être dans le visible pour réaliser des images en couleur de la rétine. Des longueurs d'onde dans le bleu ou le proche ultraviolet, typiquement entre 350 et 500 nm, peuvent également être utilisées pour la visualisation des faisceaux de fibres nerveuses dans le cade du glaucome par exemple. La source $LS_r$ est par exemple une LED ou une lampe munie d'un filtre. Le bloc illumination 11 comprend également une seconde source $LS_a$ d'illumination de la rétine destinée à l'analyse des défauts optiques du système d'imagerie. La source d'illumination $LS_a$ est une source ponctuelle, permettant de former un point source secondaire sur la rétine de l'oeil du sujet. Typiquement, la longueur d'onde de la source d'illumination $LS_a$ pour l'analyse des défauts optiques est autour de 750 nm. Une telle longueur d'onde est confortable pour le sujet et aussi proche que possible de la longueur d'onde d'imagerie. De préférence la longueur d'onde de la source $LS_a$ est différente de celle de la source $LS_r$ pour des raisons de séparation des chemins optiques entre la mesure des défauts optiques et l'imagerie de la rétine. La source $LS_a$ est par exemple une diode laser ou diode superluminescente SLD. Un ensemble de lames séparatrices $BS_1$, $BS_2$, permet d'envoyer vers l'oeil 10 du sujet les faisceaux lumineux émis par les sources $LS_r$ et $LS_a$. Un ensemble d'éléments optiques $L_2$, $L_3$, $L_4$, sont utilisées pour former à partir des sources d'illumination des faisceaux colinéaires incidents sur la pupille de l'oeil. L'image de la rétine est formée sur un plan de détection 121 du bloc détecteur 12 comprenant par exemple une caméra d'imagerie de type CCD (abréviation de l'expression anglo-saxonne « Charge Coupled Device »), au moyen d'un système d'imagerie comprenant notamment dans cet exemple un ensemble d'éléments optiques référencées $L_1$, $L_5$, $L_6$, le miroir déformable et un ensemble de lames séparatrices. Le plan de détection est dans cet exemple le plan des éléments sensibles de la caméra d'imagerie. Des systèmes 18 et 19 de type Badal permettent de compenser l'amétropie du sujet tout en assurant la conjugaison de pupille entre le plan prédéterminé de l'oeil 17 et les plans d'analyse et de correction. Le système d'imagerie présente une pupille d'entrée située dans un plan réel de l'espace d'entrée du système d'imagerie, destiné à être confondu pendant l'examen de l'oeil d'un sujet avec un plan prédéterminé 17 de l'oeil, par exemple le plan pupillaire. La pupille d'entrée est suffisamment grande pour permettre une résolution théorique suffisante, notamment pour la visualisation des cônes. Typiquement, elle est de l'ordre de 5 à 7 mm. Sur la figure 2, les plans référencés par la lettre « r » correspondent aux plans conjugués optiquement avec le plan de la rétine, tandis que les plans référencés par la lettre « p » correspondent aux plans conjugués optiquement avec ledit plan prédéterminé 17. Le dispositif d'imagerie rétinienne comprend en outre un dispositif 15 d'analyse des défauts optiques. Il s'agit d'analyser au mieux l'ensemble des perturbations que subissent les rayons lumineux entre la rétine et le détecteur. Le dispositif d'analyse des défauts optiques est par exemple un analyseur de type Shack-Hartmann (HASO® 32-eye Imagine Eyes®), comprenant un plan d'analyse formé d'un ensemble de micro lentilles et un détecteur disposé dans le plan focal des micro lentilles. Le plan d'analyse est avantageusement conjugué optiquement avec le plan 17 de la pupille d'entrée du système d'imagerie grâce à des moyens de conjugaison optique comprenant notamment les éléments optiques $L_1$, $L_5$, $L_6$, $L_7$, le miroir déformable et un ensemble de lames séparatrices. On peut définir ainsi une voie d'imagerie comprenant notamment le détecteur 12 et le système d'imagerie destiné à former l'image de la rétine sur le plan de détection du détecteur. On peut définir également une voie d'analyse comprenant notamment le dispositif 15 de mesure des défauts oculaires et les moyens de conjugaison optique du plan d'analyse avec le plan 17 de la pupille d'entrée du système d'imagerie. Le miroir déformable se trouve sur un trajet optique commun aux deux voies. Un calculateur (non représenté) permet de déterminer les défauts optiques du système et d'envoyer une commande de correction au dispositif de correction 14, par exemple un miroir déformable de type mirao 52-e Imagine Eyes®. Le plan du miroir déformable est également avantageusement conjugué optiquement avec le plan 17 de la pupille d'entrée du système d'imagerie. Un ensemble de lames séparatrices, référencées $BS_3$, $BS_4$, $BS_5$, sur la figure 2, permettent de diriger les rayons lumineux issus des sources d'illumination $LS_r$ et $LS_a$ et rétrodiffusés par la rétine sur le miroir déformable 12 puis respectivement sur le détecteur 12 et l'analyseur 15 formant respectivement les faisceaux d'imagerie et d'analyse. Selon une variante, la pupille d'entrée 17 du dispositif d'imagerie est une image de la pupille du miroir déformable.

Dans l'exemple illustré sur la figure 2, un système d'occultation, formé ici d'une pastille d'occultation 20, est positionné dans le plan de correction du dispositif de correction 14, ou à proximité du plan de correction, centrée sur l'axe optique du système d'imagerie. L'axe optique est défini généralement comme l'axe reliant le centre de la pupille d'entrée du système d'imagerie avec le centre du champ. La pastille est dimensionnée pour permettre l'occultation des réflexions des faisceaux issus des sources d'imagerie et d'analyse par la cornée.

Le plan de correction (ou un plan image de celui-ci) est un plan particulier. C'est en effet dans ce plan qu'une occultation partielle de la pupille agit de la même façon sur le faisceau d'analyse des défauts optiques et sur le faisceau d'imagerie. Plus précisément, la partie du dispositif de correction (par exemple un miroir déformable) non visible par le système de mesure des défauts optiques du fait de l'occultation du faisceau d'analyse par le système d'occultation, ne peut pas être commandée. Autrement dit, la correction qu'apporte l'élément correcteur dans cette partie n'est pas maitrisée. Il est donc important que cette partie de l'élément correcteur non maitrisée n'interagisse pas avec le faisceau d'éclairage de la rétine servant à faire l'image de la rétine quelle que soit la taille angulaire de la zone d'éclairage. Un plan « d'occultation » qui assure le respect de la contrainte ci-dessus est le plan de correction de l'élément correcteur lui-même (ou un plan très voisin) ou un plan conjugué de celui-ci dans la mesure où ce plan conjugué est situé sur une partie commune au système d'imagerie et au système de mesure des défauts optiques.

De façon avantageuse, le plan de correction sera agencé de façon à être sensiblement conjugué avec le plan image de la source d'éclairage formée par le dioptre cornéen, lors d'une imagerie de la rétine d'un sujet. Il se trouve que ce plan se trouve être très proche du plan pupillaire de l'oeil ce qui permet d'obtenir in fine un système de mesure et de correction des défauts oculaires dont les plans d'analyse et de correction se trouvent sensiblement optiquement conjugués avec le plan pupillaire de l'oeil, ce qui s'avère être une configuration avantageuse pour obtenir à la fois une bonne correction des défauts optiques sur l'axe mais aussi dans le champ d'imagerie sans générer d'effet de lucarne sur le flux rétrodiffusé par la rétine.

Alternativement, le plan de correction pourra être agencé de telle sorte à ce qu'il soit sensiblement conjugué avec le plan image de la source d'analyse par la dioptre cornéen, si les plans images par le dioptre cornéen des sources d'imagerie et d'analyse ne sont pas confondus, ou dans une position intermédiaire entre les deux plan images selon la source dont on cherche le plus à minimiser les réflexions.

Le diamètre « occultant » du système d'occultation, dans l'exemple de la figure 2 le diamètre de la pastille 20, peut être calculé de façon à être au minimum aussi grand que la taille de l'image de la source d'éclairage de la rétine formée par la cornée, multiplié par le facteur de grandissement entre le plan de l'image de la source formée par la cornée et le plan du système d'occultation. La source d'analyse étant une source quasi ponctuelle, la taille de son image par le dioptre cornéen est plus petite que celle de la source d'éclairage de la rétine, et avec un système d'occultation dimensionné pour occulter les réflexions par la cornée des faisceaux issus de la source d'imagerie, les réflexions des faisceaux issus de la source d'analyse se trouveront également occultés.

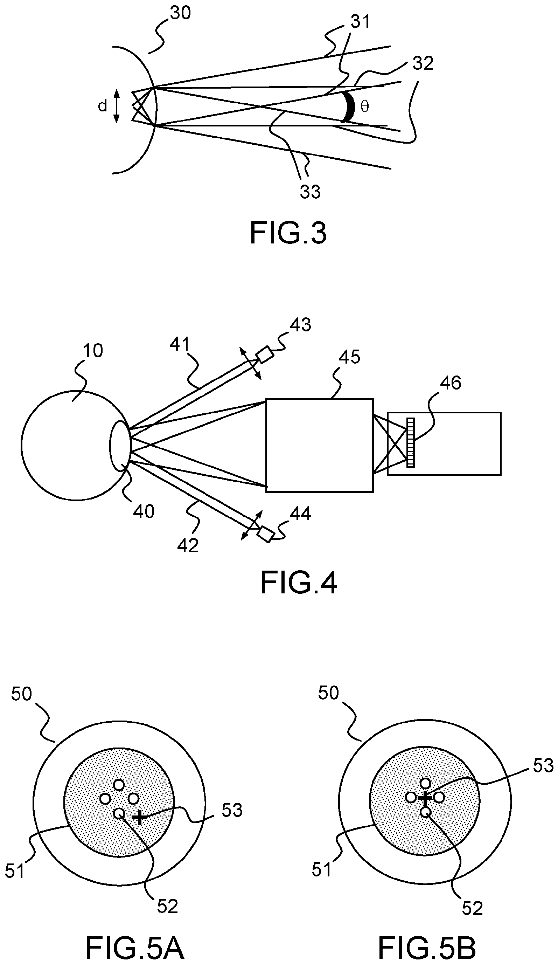

La figure 3 illustre par un schéma le calcul de la taille de l'image de la source par le dioptre cornéen dans le cas d'une source collimatée de taille angulaire θ. Les faisceaux 31, 32, 33 représentent respectivement les faisceaux de bord de champ (31, 33) et central (32). Le diamètre d de l'image est donné par d = tan θ x R/2 où R est le rayon de courbure de la cornée (R=7,8 mm en moyenne). Ainsi dans le cas d'une caméra rétinienne de champ imagé sur la rétine de 5x5°, la taille de l'image de la source d'éclairage de la rétine donnée par la formule précédente donne d = 0,340 mm. Dans ce cas, la taille de la pastille est donc déterminée de façon à ce que son image dans l'espace d'entrée du système optique d'imagerie (ou espace de sortie de l'oeil) soit au moins égale à 0,340 mm. On peut calculer que dans ce cas, le pourcentage d'occultation de la pupille est de $(0{,}340/5)^2 = 0{,}5\%$ environ, dans l'hypothèse d'une pupille d'entrée de 5 mm, ce qui comme on le verra, présente peu d'effet sur la qualité de l'image. Avantageusement, on choisira une pastille un peu plus grande pour compenser des erreurs de réalisation et/ou d'alignement du système optique. Par exemple, l'image de la pastille dans l'espace d'entrée du système d'imagerie pourra avoir un diamètre au moins égal à 0,5 mm. Dans ce cas, le pourcentage d'occultation passerait à 1%.

Dans l'exemple de la figure 2, il est prévu comme système d'occultation une pastille, Cette pastille peut par exemple être intégrée dans une lame transparente à faces planes et parallèles. D'autres systèmes sont évidemment possibles, par exemple un miroir percé qui serait placé dans un plan conjugué du plan de correction. L'occultation est en principe ronde mais elle peut être ovale dans le cas où elle serait placée sur un miroir qui travaille à incidence non nulle. Les caractéristiques de l'ovale seraient alors calculées pour obtenir une occultation ronde lorsqu'elle est projetée dans un plan perpendiculaire à l'axe optique.

Dans l'exemple de la figure 2, la pastille d'occultation est centrée sur l'axe optique du système d'imagerie. C'est une position préférée car le sommet de la cornée est en général centré sur la pupille de l'oeil que l'on cherche avantageusement à centrer sur la pupille d'entrée du système optique.

Avantageusement, le dispositif d'imagerie rétinienne selon l'invention comprend un système de positionnement spatial du système d'imagerie par rapport à l'oeil, tel que représenté sur la figure 2 (43, 44, 45, 46) ou sur la figure 4. Pour que le système d'occultation apporte tout l'effet recherché, il est important que le dispositif d'imagerie soit placé devant l'oeil à mesurer de façon à ce que l'image du système d'occultation, ramenée dans l'espace d'entrée du système optique d'imagerie, qui est l'espace de sortie de l'oeil, couvre l'image la source d'éclairage par le dioptre cornéen. Il est donc avantageux que la caméra d'imagerie rétinienne possède un système de positionnement qui permette cet ajustement. Le système de positionnement (figure 4) comprend par exemple des sources d'alignement 43, 44, couplées à une caméra de visualisation de l'oeil comprenant un objectif 45 et un détecteur 46, par exemple un détecteur matriciel. Les sources d'alignement peuvent par exemple être au nombre de 2, 3 ou 4. Elles sont imagées par le dioptre cornéen 40 de l'oeil 10. En observant la position de ces images au moyen de la caméra de visualisation de l'oeil 45, 46, on peut procéder au positionnement du dispositif.

Avantageusement, le système de positionnement pourra permettre à un opérateur ou à un logiciel de traitement d'image couplé à un système de motorisation de positionner le dispositif d'imagerie de façon à ce que les réflexions cornéennes soient occultées par le système d'occultation. Si l'opération est réalisée par l'opérateur via un joystick, l'image de la pupille de l'oeil peut inclure en overlay (en surimpression sur l'image), une petite croix par exemple symbolisant le centre de l'occultation. L'opérateur aurait dans ce cas en charge de réaliser la netteté de l'image des réflexions cornéennes liées aux sources d'alignement en ajustant la distance entre l'instrument et l'oeil du sujet et de les placer de part et d'autre de la croix en ajustant latéralement la position relative de l'instrument et l'oeil du sujet.

Les figures 5A et 5B illustrent ainsi un premier exemple (figure 5A) où la croix (référencée 53) n'est pas centrée par rapport aux images des sources d'alignement 52 (au nombre de 4 dans cet exemple) et un second exemple où l'alignement est corrigé (figure 5B). Dans ces exemples, la référence 50 représente le bord de l'iris et la référence 51 le bord de la pupille de l'oeil.

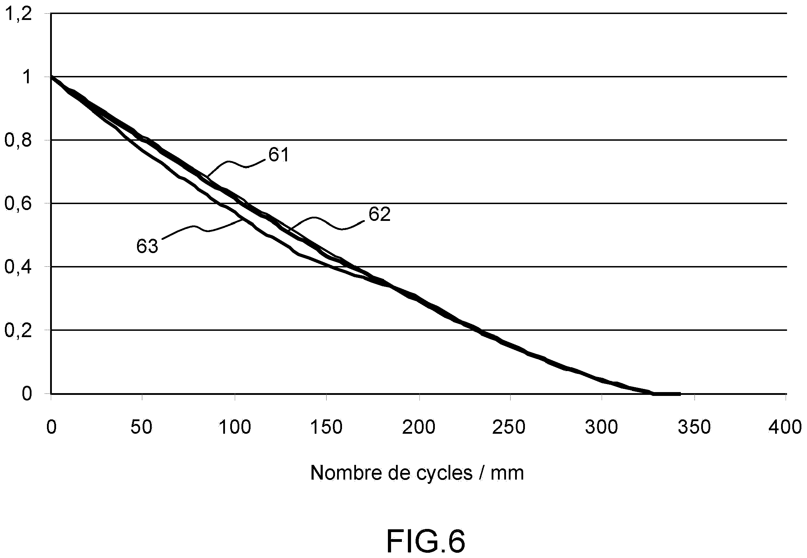

La déposante a par ailleurs démontré que l'effet de l'occultation sur la FTM était négligeable. Une simulation de FTM a été effectuée, avec et sans système d'occultation, au moyen du logiciel Zemax© et les résultats sont montrés sur la figure 6. Sur cette figure, la courbe 61 montre la FTM calculée sans occultation. La courbe 62 montre la FTM calculée avec une pastille d'occultation centrale positionnée au niveau du plan de correction, dont le diamètre de l'image ramenée dans le plan de l'image de la source d'imagerie par le dioptre cornéen est de 0,5 mm. La courbe 63 montre la FTM calculée avec une pastille d'occultation positionnée au niveau du plan de correction, dont le diamètre de l'image ramenée dans le plan de l'image de la source d'imagerie par le dioptre cornéen est de 1 mm. Ces calculs mettent en évidence que par rapport à la courbe de FTM parfaite sans occultation centrale, le fait d'avoir une occultation centrale entraine une légère baisse de la FTM en particulier sur les fréquences spatiales basses jusqu'à environ la moitié de la fréquence de coupure et entraine une légère augmentation sur les fréquences spatiales élevées jusqu'à la fréquence de coupure. Cependant, pour une occultation de 0.5 mm de diamètre dans le plan pupillaire, la variation à la hausse ou à la baisse reste complètement négligeable (moins de 1% de variation). Par ailleurs, même avec des occultations plus importantes (jusqu'à 1.3 mm), pour les fréquences spatiales élevées (au-dessus de la moitié de la fréquence de coupure), la FTM reste légèrement au-dessus de la FTM parfaite sans occultation. L'impact sur la baisse de FTM ne touche que des fréquences spatiales où la FTM est déjà au-dessus de 40% ce qui n'est pas gênant pour l'imagerie car le contraste est encore très bon pour les fréquences spatiales touchées par cette baisse de FTM.

La déposante a par ailleurs démontré que la perte de flux sur l'image formée sur la caméra est égale en pourcentage au carré du rapport entre le diamètre occultant du système d'occultation ramené dans l'espace d'entrée du système d'imagerie et le diamètre de la pupille d'entrée, ne dépasse pas 1% dans le cas d'un diamètre de 0,5 mm et d'une pupille d'entrée de diamètre 5 mm. En revanche le rapport signal à bruit est fortement amélioré car la suppression de la réflexion cornéenne diminue le bruit de façon importante.

Il est possible d'évaluer le gain sur la qualité de l'image en fonction du gain obtenu sur le rapport signal à bruit. Expérimentalement, le signal total reçu par le détecteur en présence des réflexions cornéenne dans le cas d'un système d'imagerie de type plein champ comportant une pupille d'entrée de 5 mm disposé dans le plan pupillaire de l'oeil et disposant d'un champ d'éclairage (et d'imagerie) de 4x4° avec un faisceau d'éclairage centré sur le sommet de la cornée peut se décomposer de la manière suivante :

- 42,3% du signal détecté sur le détecteur est dû à la réflexion cornéenne. Ce signal ne comporte aucune information utile mais apporte du bruit de détection ;
- 50,7% du signal détecté sur le détecteur est dû aux couches de la rétine situées au-dessus et au-dessous de la couche des photorécepteurs. Ce signal ne comporte aucune information utile mais apporte du bruit de détection ;
- 7% du signal détecté sur le détecteur est dû à la couche des photorécepteurs que l'on cherche à imager. Ce signal constitue le signal utile.

Si T1 est le signal total détecté par le détecteur, le rapport signal à bruit SNR1 prend l'expression suivante (en négligeant le bruit de lecture du détecteur négligeable par rapport au bruit de détection du signal total qui est au minimum 5 à 10 fois supérieur):

$$SNR_1 = \frac{Signal}{Bruit} = \frac{0,07 * T_1}{\sqrt{T_1}} = 0,07 * \frac{T_1}{\sqrt{T_1}}$$

Le même calcul peut être mené en considérant l'ajout d'une occultation dont le diamètre de l'image ramené dans le plan pupillaire est de 0,5 mm. L'occultation représente 1% de la pupille d'entrée et entraine mécaniquement une baisse de 1% sur le signal utile provenant de la couche des photorécepteurs et sur le signal issus des couches de la rétine situés de part et d'autre de la couche des photorécepteurs. Du fait de la suppression de la réflexion cornéenne et de l'occultation centrale qui obscurcit 1% de la pupille, la totalité du signal reçu T2 dans cette nouvelle configuration a chuté et T2=0,57*T1. Dans cette nouvelle configuration la totalité du signal T2 reçu par le détecteur est maintenant répartie comme suit :

- 87,8% du signal détecté sur le détecteur est dû aux couches de la rétine situées au-dessus et au-dessous de la couche des photorécepteurs. Ce signal ne comporte aucune information utile mais apporte du bruit de détection ;
- 12,2% du signal détecté sur le détecteur est dû à la couche des photorécepteurs que l'on cherche à imager. Ce signal constitue le signal utile.

Le nouveau rapport signal à bruit SNR2 prend alors l'expression suivante :

$$SNR_2 = \frac{Signal}{Bruit} = \frac{0.12 * T_2}{\sqrt{T_2}} = \frac{0,069 * T_1}{\sqrt{0,57 * T_1}} = 0,092 * \frac{T_1}{\sqrt{T_1}} = 1,31 * SNR_1$$

Le gain lié à l'ajout de l'occultation centrale est donc de plus de 30%.

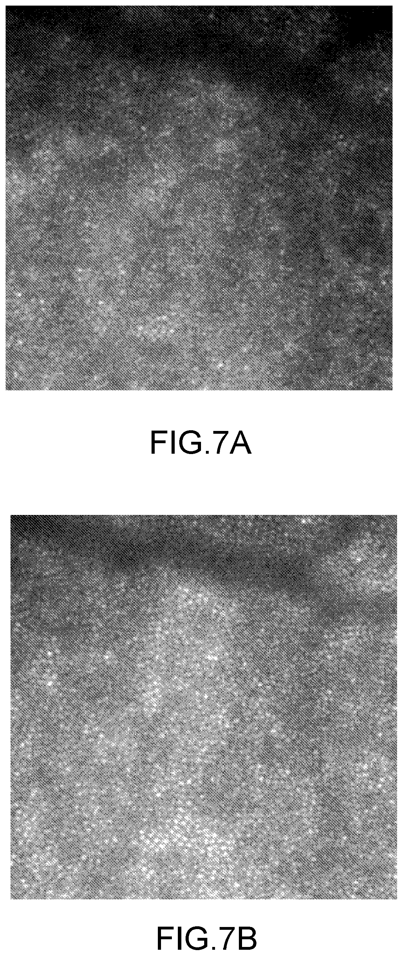

Les deux images brutes (sans traitement ni moyenage) montrées sur les figures 7A et 7B permettent d'évaluer l'apport du système d'occultation sur la qualité des images.

La première image (7A) a été réalisée avec une caméra d'imagerie plein champ à haute résolution non munie d'un système d'occultation. Le flux de l'éclairage de la rétine rentre par le sommet de la cornée et l'éclairage permettant la mesure des défauts optiques est effectué hors axe de façon à ce que les réflexions cornéennes sur cette source d'éclairage ne viennent pas perturber la mesure. Les sources d'analyse et d'éclairage sont déclenchées successivement et le système de mesure des défauts optiques est synchronisé avec la source d'éclairage d'analyse tandis que le détecteur formant l'image de la rétine est déclenché sur la source d'éclairage de la rétine.

La seconde image (7B) est réalisée avec exactement le même système utilisé pour faire la première image mais une pastille d'occultation a été rajoutée dans un plan très voisin du plan du système correcteur (c'est à dire un plan pupillaire). Le diamètre de cette occultation ramené dans l'espace de l'oeil est de 1 mm. Par ailleurs, la source d'analyse a été recentrée et rentre par le sommet de la cornée permettant un effet Styles Crawford optimal sur ce signal. Les réflexions cornéennes liées à ce signal sont bloquées par le système d'occultation et ne viennent pas perturber la mesure des défauts optiques.

L'amélioration visible sur la figure 7B est notable.

Il est à noter que cette étude est réalisée sur la réflexion cornéenne qui est un dioptre qu'il est absolument impossible d'éviter lors de l'éclairage de la rétine mais que ce même raisonnement peut s'étendre à tout dioptre situé de façon commune aux chemins optiques d'éclairage et d'imagerie.

Avantageusement, la méthode d'imagerie rétinienne selon l'invention comprend une phase de calibration, pendant laquelle le logiciel d'asservissement permettant de contrôler le dispositif de correction « apprend » comment le système de mesure des défauts optiques mesure la variation de la réponse du système correcteur lors de l'action de chacun des actionneurs pris un par un. Pour des raisons de rapidité de convergence et de qualité finale de correction, il est intéressant de réaliser la calibration de la boucle d'asservissement en passant par cette occultation centrale. Selon une variante, la calibration peut être réalisée à l'aide d'un oeil artificiel placé en lieu et place de l'oeil du sujet, auquel cas l'occultation centrale sera nécessairement en place. Selon une autre variante, on peut utiliser pour la calibration du système d'optique d'adaptative une voie spécifique de calibration comme c'est le cas par exemple dans l'exemple de la figure 2. La voie de calibration comprend une source interne $LS_c$ au foyer d'une optique $L_8$, ainsi qu'une lame séparatrice $BS_4$ permettant d'envoyer le faisceau de calibration issu de la source de calibration vers le miroir déformable 14, puis vers le dispositif 15 de mesure de défauts optiques. Dans ce cas, il faut que le chemin optique entre la source interne $LS_c$ et le système de mesure des défauts optiques passe à la fois par le système correcteur et par le système d'occultation. Bien évidemment, lorsque le système d'occultation est placé sur ou très voisin du plan de correction du système correcteur, cette contrainte est respectée. Ce qui rajoute de l'importance à placer le système d'occultation dans cette position particulière.

Bien que décrite à travers un certain nombre d'exemples de réalisation détaillés, le dispositif d'imagerie rétinienne et la méthode selon l'invention comprennent différentes variantes, modifications et perfectionnements qui apparaîtront de façon évidente à l'homme de l'art, étant entendu que ces différentes variantes, modifications et perfectionnements font partie de la portée de l'invention, telle que définie par les revendications qui suivent.

En particulier, l'invention a été décrite en prenant l'exemple d'un dispositif d'imagerie rétinienne pour lequel elle s'applique particulièrement du fait de l'importance de la réduction des réflexions cornéennes dans la voie d'imagerie, mais elle peut s'appliquer aussi aux systèmes de type AOSLO ou OCT avec optique adaptative, ou plus généralement tout dispositif d'imagerie rétinienne avec optique adaptative, dans lesquels la réduction des réflexions cornéennes dans la voie d'analyse et, dans une moindre mesure dans la voie d'imagerie, est également nécessaire pour gagner en qualité d'image.

## Revendications

1. Dispositif d'imagerie rétinienne comprenant

   - Au moins une source d'émission d'un faisceau lumineux pour l'illumination de la rétine d'un oeil d'un sujet,
   - une voie d'imagerie de la rétine comprenant un dispositif (12) de détection avec un plan de détection (121) et un système optique ($L_1$, $L_5$, $L_6$) d'imagerie,
   - une voie d'analyse comprenant un dispositif (15) de mesure de défauts optiques avec un plan d'analyse (151) destiné à recevoir un ensemble de rayons lumineux rétrodiffusés par la rétine et des moyens de conjugaison optique dudit plan d'analyse avec un plan prédéterminé dans l'espace d'entrée dudit système d'imagerie de la voie d'imagerie,
   - un dispositif (14) de correction commun auxdites voies d'analyse et d'imagerie, comprenant un plan de correction (141) et destiné à corriger dans ledit plan de correction les rayons lumineux issus de ladite source d'émission et rétrodiffusés par la rétine en fonction des défauts optiques mesurés par le dispositif de mesure des défauts optiques,
   - un système d'occultation (20) de la lumière, positionné dans un plan voisin ou confondu avec ledit plan de correction ou dans un plan image dudit plan de correction situé sur un trajet optique commun des voies d'analyse et d'imagerie, et dimensionné pour occulter au moins partiellement les réflexions par la dioptre cornéen des rayons lumineux issus de ladite source d'émission.

2. Dispositif selon la revendication 1, dans lequel ledit plan de correction est conjugué avec ledit plan prédéterminé dans l'espace d'entrée du système d'imagerie de la voie d'imagerie.

3. Dispositif selon la revendication 2, dans lequel ledit plan prédéterminé est le plan de la pupille d'entrée dudit système d'imagerie.

4. Dispositif selon l'une quelconque des revendications précédentes, dans lequel le système d'occultation présente la forme d'un disque opaque, dont le diamètre, ramené dans l'espace d'entrée du système d'imagerie, est supérieur ou égal à une valeur d donnée par $d = \tan\theta \times R/2$ où R est le rayon de courbure de la cornée et $\theta$ la taille angulaire de la source d'émission.

5. Dispositif selon l'une quelconque des revendications précédentes, dans lequel le système d'occultation est centré sur l'axe optique dudit système optique d'imagerie.

**6.** Dispositif selon l'une quelconque des revendications précédentes, dans lequel le dispositif de correction comprend un miroir déformable.

**7.** Dispositif selon l'une des revendications précédentes, dans lequel le dispositif de mesure des défauts optiques (15) est un analyseur de type Shack-Hartmann.

**8.** Dispositif selon l'une des revendications précédentes comprenant en outre un système de positionnement dans l'espace (43, 44, 45, 46) du dispositif d'imagerie par rapport à l'oeil.

**9.** Dispositif selon l'une des revendications précédentes, dans lequel le dispositif d'imagerie rétinienne est de type plein champ, comprenant une première source lumineuse d'imagerie ($LS_r$) pour l'illumination de la rétine avec un champ donné et une seconde source lumineuse d'analyse ($LS_a$) pour l'illumination de la rétine aux fins de l'analyse des défauts optiques par ledit dispositif de mesure des défauts optiques, le dispositif de détection comprenant un détecteur matriciel et le système d'occultation étant dimensionné pour occulter au moins partiellement les réflexions par le dioptre cornéen des rayons lumineux issus desdites sources.

**10.** Méthode d'imagerie rétinienne, comprenant

- l'émission d'au moins un faisceau lumineux pour l'illumination de la rétine d'un oeil (10) d'un sujet au moyen d'une source d'émission lumineuse,
- la formation d'une image d'au moins une partie de la rétine sur un plan de détection (121) d'un dispositif de détection (12), au moyen d'un système optique d'imagerie définissant une voie d'imagerie,
- la mesure de défauts optiques par l'analyse dans un plan d'analyse donné des défauts optiques de rayons lumineux rétrodiffusés par la rétine, ledit plan d'analyse étant conjugué avec un plan prédéterminé (17) de l'oeil au moyen d'un système de conjugaison optique définissant une voie d'analyse,
- la correction dans un plan de correction donné des rayons lumineux issus de ladite source d'émission et rétrodiffusés par la rétine en fonction des défauts optiques mesurés,
- l'occultation au moins partielle des réflexions par le dioptre cornéen des rayons lumineux issus de ladite source d'émission par un système d'occultation du flux lumineux agencé dans un plan voisin du plan de correction ou dans un plan conjugué du plan de correction situé sur un trajet commun aux voies d'analyse et d'imagerie.

**11.** Méthode d'imagerie rétinienne selon la revendication 11, dans laquelle le plan de correction est conjugué avec ledit plan prédéterminé de l'oeil.

**12.** Méthode d'imagerie rétinienne selon la revendication 11, dans laquelle ledit plan prédéterminé de l'oeil est sensiblement confondu avec le plan image de ladite source d'émission lumineuse par le dioptre cornéen.

**13.** Méthode d'imagerie rétinienne selon l'une quelconque des revendications 10 à 12, dans laquelle ledit plan prédéterminé de l'oeil est sensiblement confondu avec le plan de la pupille d'entrée du système d'imagerie.

**14.** Méthode selon l'une quelconque des revendications 10 à 13, dans lequel les faisceaux issus de ladite source d'émission lumineuse sont incidents dans l'oeil par le sommet de la cornée de l'oeil.

**15.** Méthode d'imagerie rétinienne selon l'une quelconque des revendications 10 à 14, comprenant en outre le positionnement dans l'espace dudit système d'imagerie par rapport à l'oeil.

**16.** Méthode d'imagerie rétinienne plein champ selon l'une quelconque des revendications 10 à 15, comprenant l'émission d'un premier faisceau lumineux d'imagerie pour l'illumination de la rétine avec un champ donné et l'émission d'un second faisceau lumineux pour l'illumination de la rétine pour l'analyse des défauts optiques et l'occultation au moins partielle des réflexions par le dioptre cornéen desdits faisceaux lumineux d'imagerie et d'analyse par ledit système d'occultation.

**Patentansprüche**

**1.** Vorrichtung zur Abbildung der Retina, umfassend

- mindestens eine Lichtstrahlemissionsquelle zum Beleuchten der Retina eines Auges eines Subjekts,

- einen Abbildungskanal für die Retina, der eine Detektionsvorrichtung (12) mit einer Detektionsebene (121) und ein optisches Abbildungssystem ($L_1$, $L_5$, $L_6$) umfasst,
- einen Analysekanal, welcher eine Vorrichtung (15) zum Messen von optischen Defekten mit einer Analyseebene (151), welche dazu geeignet ist, einen Satz von an der Retina rückgestreuten Lichtstrahlen zu empfangen, und Mittel zum optischen Konjugieren der Analyseebene zu einer vorbestimmten Ebene im Eintrittsraum des Abbildungssystems des Abbildungskanals umfasst,
- eine Korrekturvorrichtung (14), die dem Analyse- und dem Abbildungskanal gemeinsam ist, welche eine Korrekturebene (141) umfasst und dazu geeignet ist, in der Korrekturebene die von der Emissionsquelle ausgehenden und an der Retina rückgestreuten Lichtstrahlen basierend auf von der Vorrichtung zum Messen von optischen Defekten gemessenen optischen Defekte zu korrigieren,
- ein Lichtverdeckungssystem (20), das in einer zu der Korrekturebene benachbarten Ebene oder in der Korrekturebene oder in einer Bildebene der Korrekturebene, die auf einem dem Analysekanal und dem Abbildungskanal gemeinsamen optischen Weg liegt, angeordnet ist und so dimensioniert ist, dass die Reflektionen der von der Emissionsquelle ausgehenden Lichtstrahlen an der Hornhautoberfläche zumindest teilweise verdeckt werden.

2. Vorrichtung nach Anspruch 1, wobei die Korrekturebene zu der vorbestimmten Ebene im Eintrittsraum des Abbildungssystems des Abbildungskanals konjugiert ist.

3. Vorrichtung nach Anspruch 2, wobei die vorbestimmte Ebene die Ebene der Eintrittspupille des Abbildungssystems ist.

4. Vorrichtung nach einem der vorhergehenden Ansprüche, wobei das Verdeckungssystem die Form einer undurchsichtigen Scheibe hat, deren Durchmesser, übertragen in den Eintrittsraum des Abbildungssystems, größer als oder gleich einem Wert d ist, welcher gegeben ist durch d = tan $\Theta$ x R/2, wobei R der Krümmungsradius der Hornhaut und $\Theta$ die Winkelgröße der Emissionsquelle ist.

5. Vorrichtung nach einem der vorhergehenden Ansprüche, wobei das Verdeckungssystem auf die optische Achse des optischen Abbildungssystems zentriert ist.

6. Vorrichtung nach einem der vorhergehenden Ansprüche, wobei die Korrekturvorrichtung einen verformbaren Spiegel umfasst.

7. Vorrichtung nach einem der vorhergehenden Ansprüche, wobei die Vorrichtung (15) zum Messen von optischen Defekten ein Analysegerät vom Typ Shack-Hartmann ist.

8. Vorrichtung nach einem der vorhergehenden Ansprüche, ferner umfassend ein System zum Positionieren der Abbildungsvorrichtung im Raum (43, 44, 45, 46) relativ zum Auge.

9. Vorrichtung nach einem der vorhergehenden Ansprüche, wobei die Vorrichtung zur Abbildung der Retina vom Typ Vollfeld ist und eine erste Abbildungslichtquelle ($LS_r$) zum Beleuchten der Retina mit einem gegebenen Bereich und eine zweite Analyselichtquelle ($LS_a$) zum Beleuchten der Retina zum Zweck der Analyse der optischen Defekte durch die Vorrichtung zum Messen von optischen Defekten umfasst, wobei die Detektionsvorrichtung einen Matrixdetektor umfasst und das Verdeckungssystem so dimensioniert ist, dass die Reflektionen der von den Quellen ausgehenden Lichtstrahlen an der Hornhautoberfläche zumindest teilweise verdeckt werden.

10. Verfahren zur Abbildung der Retina, umfassend:

- das Emittieren mindestens eines Lichtstrahls zum Beleuchten der Retina eines Auges (10) eines Subjekts mittels einer Lichtemissionsquelle,
- das Erzeugen eines Bildes von wenigstens einem Teil der Retina auf einer Detektionsebene (121) einer Detektionsvorrichtung (12) mittels eines optischen Abbildungssystems, das einen Abbildungskanal definiert,
- das Messen von optischen Defekten durch Analysieren optischer Defekte von an der Retina rückgestreuten Lichtstrahlen in einer gegebenen Analyseebene, wobei die Analyseebene zu einer vorbestimmten Ebene (17) des Auges mittels eines Systems zum optischen Konjugieren konjugiert wird, das einen Analysekanal definiert,
- das Korrigieren von von der Emissionsquelle ausgehenden und an der Retina rückgestreuten Lichtstrahlen in einer gegebenen Korrekturebene basierend auf gemessenen optischen Defekten,
- das wenigstens teilweise Verdecken von Reflektionen von von der Emissionsquelle ausgehenden Lichtstrahlen an der Hornhautoberfläche durch ein System zum Verdecken des Lichtflusses, welches in einer zu der Korrekturebene benachbarten Ebene oder in einer zu der Korrekturebene konjugierten Ebene, die auf einem dem Analysekanal und dem Abbildungskanal gemeinsamen Weg liegt, angeordnet ist.

11. Verfahren zur Abbildung der Retina nach Anspruch 11, wobei die Korrekturebene zu der vorbestimmten Ebene des Auges konjugiert ist.

12. Verfahren zur Abbildung der Retina nach Anspruch 11, wobei die vorbestimmte Ebene des Auges im Wesentlichen mit der Bildebene der Lichtemissionsquelle auf der Hornhautoberfläche zusammenfällt.

13. Verfahren zur Abbildung der Retina nach einem der Ansprüche 10 bis 12, wobei die vorbestimmte Ebene des Auges im Wesentlichen mit der Ebene der Eintrittspupille des Abbildungssystems zusammenfällt.

14. Verfahren nach einem der Ansprüche 10 bis 13, wobei die von der Lichtemissionsquelle ausgehenden Strahlen durch den Scheitelpunkt der Hornhaut des Auges in das Auge einfallen.

15. Verfahren zur Abbildung der Retina nach einem der Ansprüche 10 bis 14, ferner umfassend räumliches Positionieren des Abbildungssystems relativ zu dem Auge.

16. Vollfeld-Retinaabbildungsverfahren nach einem der Ansprüche 10 bis 15, umfassend das Emittieren eines ersten Abbildungslichtstrahls zum Beleuchten der Retina mit einem gegebenen Bereich und das Emittieren eines zweiten Lichtstrahls zum Beleuchten der Retina zur Analyse von optischen Defekten und das wenigstens teilweise Verdecken von Reflektionen des Abbildungslichtstrahls und des Analyselichtstrahls an der Hornhautoberfläche durch das Verdeckungssystem.

**Claims**

1. A retinal imaging device comprising:

   - at least one source for emitting a light beam for illuminating the retina of an eye of a subject;
   - an imaging channel for imaging the retina, comprising a detecting device (12) with a detecting plane (121), and an imaging optical system ($L_1$, $L_5$, $L_6$);
   - an analysis channel, comprising a device (15) for measuring optical defects, with an analysis plane (151) intended to receive a set of light rays backscattered by the retina, and means for optically conjugating said analysis plane with a preset plane in the entrance space of said imaging system of the imaging channel;
   - a correcting device (14) common to said analysis and imaging channels, comprising a correcting plane (141), and intended to correct, in said correcting plane, the light rays originating from said emitting source and backscattered by the retina, depending on optical defects measured by the device for measuring optical defects; and
   - a system (20) for blocking light, positioned in a plane neighboring or coincident with said correcting plane, or in an image plane of said correcting plane located on an optical path common to the analysis and imaging channels, and dimensioned to at least partially block reflection of light rays originating from said emitting source from the corneal diopter.

2. The device as claimed in claim 1, in which said correcting plane is conjugated with said preset plane in the entrance space of the imaging system of the imaging channel.

3. The device as claimed in claim 2, in which said preset plane is the plane of the entrance pupil of said imaging system.

4. The device as claimed in any one of the preceding claims, in which the blocking system takes the form of an opaque disk the diameter of which, projected into the entrance space of the imaging system, is greater than or equal to a value d given by $d = \tan \theta \times R/2$, where R is the radius of curvature of the cornea and $\theta$ is the angular diameter of the emitting source.

5. The device as claimed in any one of the preceding claims, in which the blocking system is centered on the optical axis of said imaging optical system.

6. The device as claimed in any one of the preceding claims, in which the correcting device comprises a deformable mirror.

7. The device as claimed in one of the preceding claims, in which the device (15) for measuring optical defects is a Shack-Hartmann analyzer.

8. The device as claimed in one of the preceding claims, furthermore comprising a system (43, 44, 45, 46) for positioning the imaging device in space relative to the eye.

9. The device as claimed in one of the preceding claims, in which the retinal imaging device is of the full-field type, comprising an imaging first light source ($LS_r$) for illuminating a given field of the retina, and an analysis second light source ($LS_a$) for illuminating the retina for the purpose of analyzing optical defects with said device for measuring optical defects, the detecting device comprising a matrix detector and the blocking system being dimensioned to at least partially block reflection of the light rays originating from said sources from the corneal diopter.

10. A retinal imaging method, comprising:

   - emitting at least one light beam in order to illuminate the retina of an eye (10) of a subject, by means of light-emitting source;
   - forming an image of at least one part of the retina on a detecting plane (121) of a detecting device (12), by means of an imaging optical system defining an imaging channel;
   - measuring optical defects by analyzing, in a given analysis plane, optical defects in light rays backscattered by the retina, said analysis plane being conjugated with a preset plane (17) of the eye by means of an optically conjugating system defining an analysis channel;
   - correcting, in a given correcting plane, light rays originating from said emitting source and backscattered by the retina, depending on the measured optical defects; and
   - at least partially blocking reflection of light rays originating from said emitting source from the corneal diopter using a system for blocking light flux, said system being arranged in a plane neighboring the correcting plane, or in a plane conjugated with the correcting plane located on a path common to the analysis and imaging channels.

11. The retinal imaging method as claimed in claim 11, in which the correcting plane is conjugated with said preset plane of the eye.

12. The retinal imaging method as claimed in claim 11, in which said preset plane of the eye is substantially coincident with the image plane of said light-emitting source formed by the corneal diopter.

13. The retinal imaging method as claimed in any one of claims 10 to 12, in which said preset plane of the eye is substantially coincident with the plane of the entrance pupil of the imaging system.

14. The method as claimed in any one of claims 10 to 13, in which the beams originating from said light-emitting source are incident into the eye via the apex of the cornea of the eye.

15. The retinal imaging method as claimed in any one of claims 10 to 14, furthermore comprising positioning said imaging system in space relative to the eye.

16. The full-field retinal imaging method as claimed in any one of claims 10 to 15, comprising emitting an imaging first light beam in order to illuminate a given field of the retina, and emitting a second light beam for illuminating the retina for the purpose of analyzing optical defects, and at least partially blocking reflection of said imaging and analysis light beams from the corneal diopter using said blocking system.

FIG.1

FIG.2

FIG.3

FIG.4

FIG.5A

FIG.5B

FIG.6

EP 2 677 920 B1

FIG.7A

FIG.7B

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 3594071 A
- WO 2010128651 A
- WO 2010079550 A
- US 20070258045 A

**Littérature non-brevet citée dans la description**

- **A. ROORDA et al.** Adaptive optics scanning laser ophthalmoscopy. *Optics express 405,* 2002, vol. 10 (9
- **R. ZAWADZKI.** Adaptive-optics optical coherence tomography for high resolution and high speed 3D retinal in vivo imaging. *Optics Express 8532,* 2005, vol. 13 (21
- **H. HOFER et al.** Improvements in retinal image quality with dynamic correction of the eye's aberrations. *Optics Express,* 2001, vol. 8 (11), 631-643
- **A. ROORDA.** Adaptive Optics Ophthalmoscopy. *Journal of refractive Surgery,* 2000, vol. 16